# EUROPÄISCHE PATENTSCHRIFT

(11) **EP 2 052 755 B1**
(45) Veröffentlichungstag und Bekanntmachung des Hinweises auf die Patenterteilung: **01.09.2010**
(21) Anmeldenummer: 08167527.4
(22) Anmeldetag: 24.10.2008
(51) Int. Cl.: A61M 25/00

(54) **Medizinisches Instrument mit seitlich ausspreizbaren Injektionsnadeln**
Medical instrument with injection needles capable of being spread out laterally
Instrument médical doté d'aiguilles d'injection apte à être déployées latéralement

(30) Priorität: 26.10.2007 DE 102007052513
(43) Veröffentlichungstag der Anmeldung: 29.04.2009
(73) Patentinhaber: Karl Storz GmbH & Co. KG, 78532 Tuttlingen (DE)
(72) Erfinder: Maerten, Dr. Philippe, 1010 Lausanne (CH); Efinger, Andreas, 78604 Rietheim (DE); Hermle, Rainer, 78559 Gosheim (DE)
(74) Vertreter: Witte, Weller & Partner

(56) Entgegenhaltungen:
- WO-A-01/08741
- US-A- 5 419 777
- US-A1- 2004 138 621

## Beschreibung

Die Erfindung betrifft ein medizinisches Instrument, mit einem tubusförmigen Körper, in dem zumindest eine Injektionsnadel axial unverschiebbar aufgenommen ist, die einen radial nach außen gerichteten Spitzenabschnitt aufweist, und mit einem Steuerelement zum seitlichen Ausfahren zumindest der Spitze der zumindest einen Injektionsnadel aus dem tubusförmigen Körper hinaus, wobei das Steuerelement axial verschiebbar im tubusförmigen Körper aufgenommen ist, und wobei dieses derart mit der zumindest einen Injektionsnadel in Wirkverbindung steht, dass in einer ersten axialen Verschiebeposition der nach außen gerichtete Spitzenabschnitt der zumindest einen Injektionsnadel durch das Steuerelement vollständig in den tubusförmigen Körper eingezogen ist, und wobei in einer zweiten axialen Verschiebeposition zumindest die Spitze des nach außen gerichteten Spitzenabschnittes durch das Steuerelement seitlich aus dem tubusförmigen Körper herausgedrückt ist.

Ein derartiges medizinisches Instrument ist aus der US-A-5,419,777 bekannt.

Das Steuerelement weist einen etwa stabförmigen Körper auf, an dessen Außenseite Führungsnuten eingeschnitten sind, in die jeweils eine Injektionsnadel eingelegt ist.

Am distalen Ende weist jede Nut eine von radial innen nach radial außen ansteigende Nockenfläche auf. Die Injektionsnadeln sind zumindest im Bereich der distalen Spitzenabschnitte etwas radial nach außen gebogen. Die Injektionsnadeln als solche sind axial unverschieblich, können jedoch radial im Bereich ihres Spitzenabschnittes nach außen herausgedrückt werden.

Dies erfolgt wenn das Steuerelement von distal nach proximal bewegt wird. Dabei trifft die nach außen ansteigende Nockenfläche der Nut auf den leicht vorgekrümmten Spitzenabschnitt einer Injektionsnadel und schiebt sie dabei radial nach außen.

Bei der entgegengesetzen Bewegung wird, wenn sich die ansteigende Nockenfläche vom Spitzenabschnitt der Injektionsnadel löst, dieser die Möglichkeit eröffnet, wieder radial nach innen in die Nut einzutreten.

Hieran ist nachteilig, dass nicht mit absoluter Sicherheit garantiert werden kann, dass der nach außen gerichtete Spitzenabschnitt wieder vollständig in die Nut des Steuerelements hineinbewegt wird.

Dies deswegen, da die Spitze einer Nadel in ein Gewebe eines Gefäßes eingestochen sein kann und dort relativ fest gehalten ist.

Dies kann fatale Folgen beim Abziehen des medizinischen Instrumentes aus dem Gefäß haben, nämlich, dass dann über die gesamte Abziehlänge durch die noch über die Außenseite des tubusförmigen Körpers hinaussehende Spitze das Gefäß verletzt oder gar eingeschnitten werden kann.

Aus der WO 01/08741 ist ein Katheterpositionierungssystem bekannt. Bei diesem Positionierungssystem sind elastische drahtförmige Finger aufgenommen, die einen radial nach außen gebogenen Endabschnitt aufweisen. In einer ersten zurückgezogenen Position eines Steuerelementes bewegen sich die Finger aufgrund deren Elastizität soweit radial nach innen in den Katheter, dass sie in diesen vollständig eintreten. Wird das Steuerelement axial verschoben, drückt es die elastischen Finger seitlich aus dem Katheter heraus, so dass dieser dann entsprechend verankert bzw. positioniert werden kann.

Da hier das Zurückkehren der Finger aufgrund deren Elastizität erfolgt, kann auch hier nicht ausgeschlossen werden, falls ein solcher Finger relativ fest im Gewebe steckt, dass dessen elastische Rückstellkraft nicht ausreicht, dass der Finger wieder vollständig in das Kathetergehäuse eingezogen wird.

Dann sind die zuvor beschriebenen fatalen Folgen ebenfalls zu beobachten.

Aus der US-A-5,538,504 ist bekannt, das Steuerelement als aufblähbaren Ballon auszubilden, der beim Aufblähen den Spitzenabschnitt der Injektionsnadel radial nach außen drückt. Die Rückstellung nach Ablassen des Blähdruckes erfolgt durch die Elastizität der Injektionsnadel.

Es ist Aufgabe der vorliegenden Erfindung ein medizinisches Instrument der eingangs genannten Art dahingehend weiterzuentwickeln, dass über eine einfache handhabbare Steuerung sichergestellt ist, dass ein nach außen gerichteter Spitzenabschnitt der zumindest einen Injektionsnadel wieder in die vollständig eingezogene Position bewegt wird.

Erfindungsgemäß wird die Aufgabe dadurch gelöst, dass das Steuerelement ein Einziehelement aufweist, das beim axialen Verschieben des Steuerelements von der zweiten in die erste Position den Spitzenabschnitt der zumindest einen Injektionsnadel wieder vollständig in den tubusförmigen Körper einzieht.

Diese Maßnahme hat den Vorteil, dass ein spezielles Einziehelement vorgesehen ist, dass derart mit der Injektionsnadel in Wirkverbindung steht, dass ganz definiert durch dieses Einziehelement sichergestellt ist, dass der seitlich ausgedrückte Spitzenabschnitt wieder eingezogen wird.

In einer weiteren Ausgestaltung weist das Steuerelement ein Spreizelement auf, das beim axialen Verschieben des Steuerelements von der ersten in die zweite Verschiebeposition den Spitzenabschnitt der Injektionsnadel seitlich über den tubusförmigen Körper hinausdrückt.

Diese Maßnahme hat den Vorteil, dass über dieses Spreizelement definiert ein Eingriffskörper geschaffen werden kann, der für die Herausdrückbewegung entsprechend ausgestaltet und geeignet ist.

Dazu wird in einer weiteren Ausgestaltung vorgeschlagen, dass das Spreizelement als Spreizkeil, bzw. das Einziehelement als Einziehkeil, ausgebildet ist.

Diese Maßnahme hat den Vorteil, dass durch mechanisch einfache Art und Weise die axiale, also lineare Hin- und Herbewegung des Steuerelementes über die Keilfläche in eine radial nach außen gerichtete Spreizbewegung bzw. wieder in eine radial nach innen gerichtete Einziehbewegung an der Injektionsnadel umgesetzt werden kann. Durch Vorsehen von Keilen mit Keilflächen ist keine mechanisch feste Verbindung zwischen dem Steuerelement und der jeweiligen Injektionsnadel notwendig, sondern es reicht aus, wenn die Keilflächen an entsprechenden Stellen an der Injektionsnadel vorbeigleiten, um die Bewegung zu steuern.

Dies eröffnet auch die Möglichkeit, diese beiden Teile nach einem Gebrauch einfach voneinander zu trennen und zu reinigen. Die Ausgestaltung und insbesondere die Steigung der Keile bzw. der Keilfläche erlaubt es, unterschiedliche Ausdruck- bzw. Einziehlängen zu bewerkstelligen.

In einer weiteren Ausgestaltung der Erfindung muss das Steuerelement aus der ersten und der zweiten axialen Verschiebeposition zunächst über eine Drehbewegung in eine verdrehte Position bewegt werden, bevor es in die jeweils andere axiale Verschiebeposition bewegt wird.

Diese Maßnahme hat den Vorteil, dass die Steuerteile am Steuerelement sehr kompakt gebaut werden können und sich gegenseitig nicht behindern. Das bedeutet, dass das Spreizelement bzw. der Spreizkeil nicht in der direkten Verschiebebahn des Einziehelements bzw. des Einziehkeils liegt, diese sich also nicht gegenseitig behindern.

In einer weiteren Ausgestaltung ist dazu vorgesehen, dass die Keilflächen von Spreizkeil und Einziehkeil umfänglich versetzt sind.

Diese Maßnahme hat den Vorteil, dass diese beiden Keile axial sehr nah voneinander beabstandet sein können und somit die Vorrichtung sehr kompakt baut. In der einen Drehstellung tritt nur der eine Keil mit der Injektionsnadel in Wirkverbindung, in der anderen Drehstellung dann entsprechend nur der andere.

In einer weiteren Ausgestaltung der Erfindung ist eine Zwangssteuerung vorhanden, die die Bewegung des Steuerelements derart steuert, dass eine geschlossene viereckförmige Steuerbahn vorhanden ist.

Diese Maßnahme hat den Vorteil, dass die Steuerbewegung für den Arzt vorgegeben ist. Zum Ausfahren der Spitze der Injektionsnadel führt er beispielsweise eine axiale und nach distal gerichtete Vorschubbewegung durch, wobei diese Bahn noch durch entsprechende Endrastpunkte klar definiert sein kann. Durch die folgende Drehbewegung kommt der Spreizkeil außer Eingriff mit der Injektionsnadel und der Einziehkeil wird nun in eine Position gedreht, in der dieser mit der Injektionsnadel in Wirkverbindung treten kann. Durch eine anschließend wieder axial verlaufende nach proximal gerichtete Verschiebebewegung, tritt der Einziehkeil mit der Injektionsnadel in Wirkverbindung und zieht diese ein. Wie bereits erwähnt, stören sich dann Einziehkeil und Spreizkeil durch den umfänglichen Versatz der Keilflächen nicht. Vor Durchführen einer erneuten Ausdrückbewegung wird das Spreizelement im entgegengesetzten Sinne wieder umfänglich gedreht, so dass das Spreizelement wieder an seinem Ausgangspunkt befindlich ist und für einen weiteren Ausdrückvorgang zur Verfügung steht.

In einer weiteren Ausgestaltung der Erfindung sind im tubusförmigen Körper mehrere Injektionsnadeln aufgenommen und die mehreren Injektionsnadeln sind durch ein einziges Steuerelement steuerbar.

Diese Maßnahme hat den Vorteil, dass nur ein einziges Steuerelement bereitgestellt werden muss und dass die mehreren Injektionsnadeln gleichzeitig seitlich ausgeschoben oder wieder eingezogen werden können.

In einer weiteren Ausgestaltung der Erfindung weist das Steuerelement dazu Kerben auf, in denen zumindest der nach außen gerichtete Spitzenabschnitt der Injektionsnadeln aufnehmbar ist.

Diese Maßnahme hat den Vorteil, dass insgesamt gesehen die Vorrichtung wenig raumergreifend ist, da die gekrümmten Spitzenabschnitte der Kanülen in den Kerben im Steuerelement aufgenommen sind.

In einer weiteren Ausgestaltung der Erfindung weisen die nach außen gerichteten Spitzenabschnitte der Injektionsnadeln einen ersten Abschnitt auf, der mit dem Spreizelement und einen zweiten Abschnitt, der mit dem Einziehelement in Wirkverbindung treten kann.

Diese Maßnahme hat den Vorteil, dass konstruktiv einfach die Wirkverbindung zwischen dem Steuerelement und dem gekrümmten Abschnitt der Injektionsnadel an getrennten Stellen hergestellt werden kann.

In einer weiteren Ausgestaltung der Erfindung ist der zweite Abschnitt der Injektionsnadeln als Keil ausgebildet, der mit dem Einziehkeil beim Einziehen der Injektionsnadeln in Verbindung steht.

Diese Maßnahme hat den Vorteil, dass durch Vorsehen dieses zusätzlichen Keils sichergestellt ist, dass durch Aneinandervorbeigleiten der beiden Keilflächen dieses Keils und des Einziehkeils, die nach außen gebogenen Spitzenabschnitte ganz definiert eingezogen und in eine definierte Endlage gebracht werden können, in der absolut sichergestellt ist, dass auch die Spitzen nicht mehr seitlich über den tubusförmigen Körper hinausschauen. Dies ist auch dann sichergestellt, wenn über die äußeren Öffnungen Kontaminationen wie Körperflüssigkeiten, Blut oder dergleichen in das Innere des tubusförmigen Körpers gelangt sein sollten. Selbst an den Keilflächen anhaftende Kontaminationen würden durch die beiden aneinander vorbeigleitenden Keilflächen zur Seite geschoben, so dass auch in stark kontaminiertem Zustand die Steuerung einwandfrei funktioniert.

In einer weiteren Ausgestaltung der Erfindung sind die mehreren Injektionsnadeln in einem Schutzschaft montiert.

Wie bereits erwähnt, ist keine mechanisch dauerhafte feste Verbindung zwischen dem Steuerelement und den Injektionsnadeln notwendig, da über die Spreizelemente bzw. Keile die radiale Bewegung der Injektionsnadeln gesteuert wird. Daher ist es ausreichend, wenn Steuerelement und die Injektionsnadel nach Montage in einer vordefinierten Lage zueinander angeordnet sind, ohne dass diese mechanisch im Steuerbereich untereinander verbunden sein müssen. Dies wird nun zusätzlich dadurch verbessert, dass bei Vorhandensein von mehreren Injektionsnadeln diese in einem Schutzrohr montiert sind. Somit können die Injektionsnadeln schon bei der Herstellung exakt lagedefiniert ausgerichtet werden. Dies erleichtert auch die Montage und Demontage, da die gegebenenfalls extrem dünnen und langen Injektionsnadeln durch den Schutzschaft geschützt sind.

Das Steuerelement kann von proximal in den tubusförmigen Körper eingeschoben werden.

Diese Maßnahme hat den Vorteil, dass dieses Steuerelement nach Montage, beispielsweise des Schutzschaftes in dem tubusförmigen Körper, von proximal eingeschoben und exakt in der gewünschten Lage benachbart zu den Injektionsnadeln positioniert werden kann. Dieses Einschieben des Steuerelementes ist beim Montieren sehr gut kontrollierbar. Beim Demontieren kann das Steuerelement auch einfach wieder abgezogen werden, ohne dass die Gefahr besteht, dabei eine Injektionsnadel zu beschädigen.

In einer weiteren Ausgestaltung der Erfindung ist das Steuerelement als stabförmiger Körper ausgebildet, der distal das Spreizelement und das Einziehelement trägt.

Dies hat insbesondere bei der Ausgestaltung mit mehreren Injektionsnadeln und mit dem gekerbten Körper den Vorteil, dass der stabförmige Körper von proximal so in den tubusförmigen Körper eingeschoben werden kann, dass die mehreren Injektionsnadeln exakt ausgerichtet in die Kerben eintreten können. Dies erleichtert insbesondere die Handhabe bei der Montage und der Demontage.

Der stabförmige Körper kann einen Steuergriff aufweisen, der proximal vom stabförmigen Körper abgewinkelt ist.

Diese Maßnahme hat den Vorteil, dass anhand des Steuergriffs die Steuerung bzw. die Bewegung des Steuerelementes durchgeführt werden kann. Die Handhabungsperson kann den Steuergriff ergreifen und das Steuerelement durch Manipulationen in die unterschiedlichen Verschiebepositionen bzw. Drehpositionen bringen, wobei das durch die Zwangssteuerung noch erheblich erleichtert ist.

In einer weiteren Ausgestaltung der Erfindung ist der stabförmige Körper von proximal in den Schutzschaft mittig zwischen die mehreren Injektionsnadeln einschiebbar.

Diese Maßnahme hat, wie zuvor erwähnt, den besonderen Vorteil bei der Montage und der Demontage bei mehreren Injektionsnadeln. Beim Montieren kann zunächst der Schutzschaft mit den mehreren Injektionsnadeln in den tubusförmigen Körper eingeschoben und positioniert werden. Anschließend wird dann der stabförmige Körper von proximal exakt lagepositioniert zwischen die Injektionsnadeln gebracht.

In einer Ausgestaltung ist das medizinische Instrument als Anuskop ausgebildet.

Diese Maßnahme hat den Vorteil, dass insbesondere für diesen Anwendungsbereich die Erfindung besonders geeignet ist, da damit entsprechende Injektionsbehandlungen ggf. auch an mehreren axial voneinander beabstandeten Stellen durchgeführt werden kann, wobei sichergestellt ist, dass beim Abziehen oder axialen Verschieben keine Verletzungen über vorstehende Injektionsnadelspitzen erfolgen können.

Es versteht sich, dass die vorstehend genannten und die nachstehend noch zu erläuternden Merkmale nicht nur in der jeweils angegebenen Kombination, sondern auch in anderen Kombinationen oder in Alleinstellung einsetzbar sind, ohne den Rahmen der vorliegenden Erfindung zu verlassen.

Die Erfindung wird nachfolgend anhand eines ausgewählten Ausführungsbeispiels in Zusammenhang mit den beiliegenden Zeichnungen näher beschrieben und erläutert. Es zeigen:
- Fig. 1: eine perspektivische Ansicht eines Bauelementes der medizinischen Vor- richtung, nämlich der tubusförmige Körper, von dem seitlich ein Handgriff vorsteht,
- Fig. 2: eine perspektivische Ansicht eines weiteren Bauteiles, nämlich ein Schutz- rohr, in dem vier Injektionsnadeln aufgenommen sind,
- Fig. 3: eine entsprechende perspektivische Darstellung eines weiteren Bauelemen- tes der erfindungsgemäßen medizinischen Vorrichtung, nämlich das Steu- erelement mit dem proximalseitigen Steuergriff,
- Fig. 4: das medizinische Instrument im zusammengebauten Zustand, wobei zunächst von proximal in den tubusförmigen Körper der Schutzschaft von Fig. 2 eingeschoben wurde und anschließend das aus Fig. 3 ersichtliche Steuerelement von proximal in den Schutzschaft eingeschoben wurde, und zwar so weit, dass die Spitzen der Injektionsnadeln noch nicht seitlich her- ausgedrückt sind,
- Fig. 5: eine zweite Verschiebestellung, in der das Steuerelement axial nach distal verschoben ist und dadurch die Spitzen seitlich über den tubusförmigen Körper hinausgedrückt sind,
- Fig. 6: eine weitere Drehstellung des Steuerelements bevor dieses wieder axial abgezogen wird,
- Fig. 7: einen Längsschnitt im Bereich des distalen Endes der medizinischen Vor- richtung in einer Position, wie sie in Fig. 4 dargestellt ist,
- Fig. 8: den entsprechenden Längsschnitt, der der Situation von Fig. 5 entspricht,
- Fig. 9: einen entsprechenden Längsschnitt, der der Situation von Fig. 6 entspricht,
- Fig. 10: eine der Fig. 9 vergleichbare Darstellung, wobei das Steuerelement so weit zurückgezogen ist, dass dessen Einziehkeil gerade mit dem Keil am ge- krümmten Spitzenabschnitt der Injektionsnadel in Eingriff kommt,
- Fig. 11: eine der Fig. 10 entsprechende Darstellung, bei vollständig zurückgezoge- nem Steuerelement und dadurch vollkommen eingezogenem Spitzenab- schnitt einer Injektionsnadel, und
- Fig. 12: wieder die Ausgangssituation nach Verdrehen des Steuerelements um die Längsachse, wobei diese Ausgangssituation wieder der Situation von Fig. 7 bzw. Fig. 4 entspricht.

Ein in den Figuren dargestelltes medizinisches Instrument ist in seiner Gesamtheit mit der Bezugsziffer 10 bezeichnet.

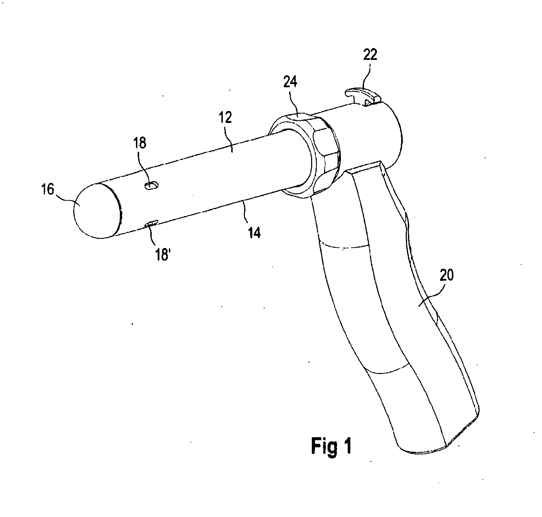

Aus Fig. 1 ist ersichtlich, dass das medizinische Instrument einen langerstreckten tubusförmigen Körper 12 in Form eines Rohres 14 aufweist, das distalseitig über eine halbrunde Kappe 16 abgeschlossen ist. Länge und Durchmesser des Rohres 14 sind so ausgebildet, dass es in den Anus eines menschlichen Körpers eingeschoben werden kann.

Im Bereich der endseitigen Kappe 16 sind im tubusförmigen Körper 12 umfänglich verteilt vier Öffnungen 18, 18' vorgesehen, deren Sinn und Zweck später erläutert werden.

Proximalseitig ist der tubusförmige Körper 12 mit einem seitlich schräg abstehenden Griff 20 verbunden, über den die Handhabungsperson das medizinische Instrument 10 mit einer Hand ergreifen kann. Eine Überwurfmutter 24 sorgt für eine lösbare Verbindung zwischen Griff 20 und Rohr 14. Eine Raste 22 ist dazu vorgesehen, weitere nachfolgend noch zu beschreibende Bauteile, die von proximal in das Rohr 14 eingeschoben werden, zu verrasten.

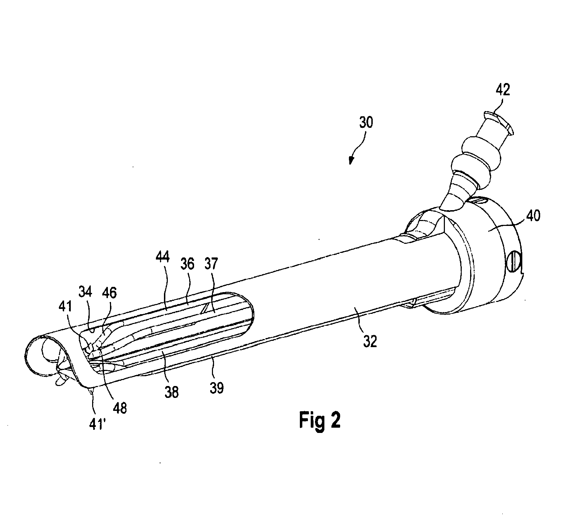

Eines dieser weiteren Bauteile ist der in Fig. 2 dargestellte Schutzschaft 30.

Der Schutzschaft 30 weist ebenfalls ein Rohr 32 auf, dessen Außendurchmesser und dessen Länge so bemessen ist, dass es in das Rohr 14 des tubusförmigen Körpers 12 passend eingeschoben werden kann.

Das Rohr 32 weist mehrere Fenster 34 auf, über die von proximal innen Einblick in den Anus bzw. in eine Körperhöhle gewährt ist.

Im Innenraum des Rohres 32 sind umfänglich gleichmäßig verteilt vier Injektionsnadeln 36, 37, 38, 39 montiert. Alle vier Injektionsnadeln 36 bis 39 sind proximalseitig an einem Flansch 40 befestigt, der mit einem LUER-Anschluss 42 versehen ist. Über den LUER-Anschluss 42 und den Flansch 40 können alle vier Injektionsnadeln 36, 37, 38, 39 mit einer Flüssigkeit beaufschlagt werden, die jeweils an einer Spitze 41, 41' austreten. Der Übersicht halber sind nur die beiden Spitzen 41, 41' der vier Injektionsnadeln 36 bis 39 bezeichnet.

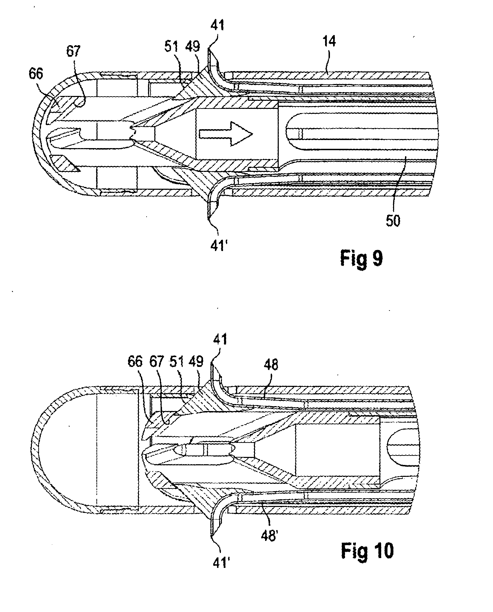

Jede der Injektionsnadeln 36 bis 39 weist eine längsverlaufende Kanüle 44 auf, die über eine radial nach innen gerichtete Krümmung 46 in einen radial nach außen gerichteten Spitzenabschnitt 48, 48' übergeht, der dann in der entsprechenden Spitze 41, 41' endet. Die nähere Ausgestaltung und der Sinn und Zweck wird später anhand der Fig. 9 bis 12 näher beschrieben und erläutert.

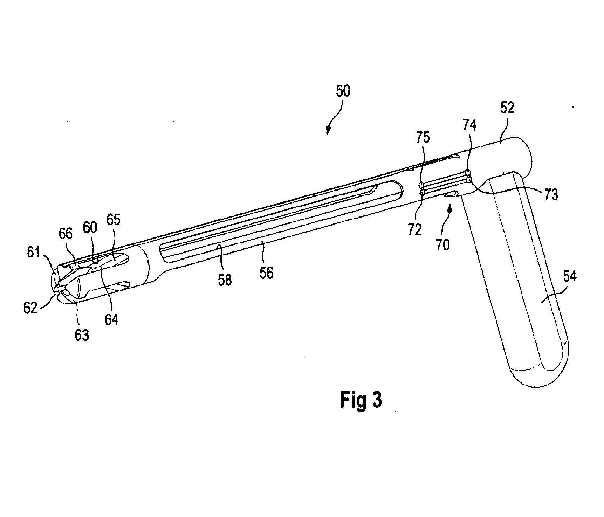

In Fig. 3 ist ein weiteres Bauteil des medizinischen Instrumentes 10 ersichtlich, nämlich das Steuerelement 50.

Das Steuerelement 50 weist einen Körper in Form eines langerstreckten Stabes 52 auf, der proximalseitig einen im dargestellten Ausführungsbeispiel seitlich abstehenden Steuergriff 54 aufweist. Über seine wesentliche Längserstreckung ist der Stab 52 als Hohlkörper 56 ausgebildet und weist mehrere Fenster bzw. Aussparungen 58 auf. Am distalen Ende weist der Körper umfänglich verteilt vier Kerben 60, 61, 62, 63 auf, die in ihrer Breite, Länge und Tiefe so ausgestaltet sind, dass darin der gekrümmte Spitzenabschnitt jeweils einer Injektionsnadel aufgenommen werden kann.

An jeder Kerbe 60, 61, 62, 63 ist proximalseitig ein Spreizkeil 64 ausgebildet, der eine Keilfläche 65 aufweist, die von proximal nach distal gesehen, radial nach innen geneigt ist. Dem Spreizkeil 64 gegenüberliegend ist ein entsprechender Einziehkeil 66, der eine entsprechende Keilfläche 67 aufweist. Die Ausgestaltung und der Sinn und Zweck der Keile werden näher in Zusammenhang mit Fig. 9 bis 12 beschrieben.

Es ist zu erwähnten, dass die Keilfläche 65 des Spreizkeiles 64 etwas umfänglich versetzt zur Keilfläche 67 des Einziehkeiles 66 ist.

In der Außenseite des Hohlkörpers 56 benachbart zum Steuergriff 54 ist eine Steuerkurve 70 eingeschnitten, die rechteckförmig ist. Diese Steuerkurve 70 weist einen ersten Rastpunkt 72, einen zweiten, proximal davon beabstandeten, Rastpunkt 73, einen gegenüber diesem umfänglich versetzten dritten Rastpunkt 74 und einen gegenüber diesem nach distal gelegenen vierten Rastpunkt 75 auf, der entsprechend umfänglich versetzt zum ersten Rastpunkt 72 liegt. An der Innenseite des Schutzschaftes 30 im Bereich des Flansches 40, springt ein entsprechendes federbelastetes stiftförmiges Rastelement vor, das in die Steuerkurve 70 eingreift.

Bei der Montage des medizinischen Instrumentes 10 wird zunächst der Schutzschaft von Fig. 2 von proximal in den tubusförmigen Körper 12 eingeschoben und über die Raste 22 lagegerecht verrastet. Dabei kommen die vier Spitzen 41, 41' der Injektionsnadeln 36 bis 39 im Bereich der Öffnungen 18, 18' zum Liegen. Anschließend wird das aus Fig. 3 ersichtliche Steuerelement 50 von proximal in den bereits im tubusförmigen Körper 12 montierten Schutzschaft 30 eingeschoben.

Dabei wird das Steuerelement 50 so eingeschoben, dass es sich in den Käfig aus den vier umfänglich verteilten Injektionsnadeln 36 bis 39 mittig hineinschiebt, und zwar derart, dass die gekrümmten Spitzenabschnitte 48, 48' und die Krümmung 46 in den Kerben 60 bis 63 zum Liegen kommen. Die Ausrichtung ist dann so, dass das Steuerelement 50 mit der zuvor beschriebenen Raste im ersten Rastpunkt 72 verrastet.

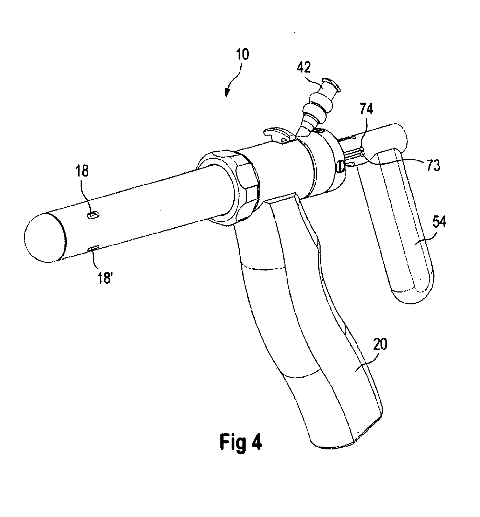

Diese endmontierte Stellung ist in Fig. 4 dargestellt.

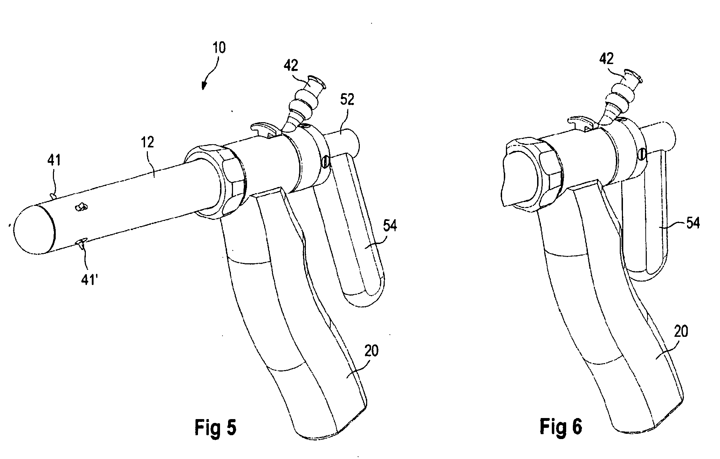

Zum seitlichen Ausspreizen wird das Steuerelement 50 von proximal nach distal so weit verschoben, bis die Raste am Rastpunkt 73 einrastet. Diese Situation ist in Fig. 5 dargestellt. Dabei sind dann die Keilflächen 65 des Spreizkeiles 64 jeweils mit der Krümmung 46 einer Injektionsnadel 36 bis 39 in Berührung getreten und haben diese radial nach außen herausgedrückt, und zwar so weit, dass deren Spitzen 41, 41' radial über die Außenseite bzw. Außenfläche des tubusförmigen Körpers 12 hinausreichen. Nunmehr kann über den LUER-Anschluss 42 ein Medikament über die Injektionsnadel an vier umfänglich versetzten Stellen, beispielsweise im Rektum, appliziert werden, wenn das medizinische Instrument als Anuskop ausgebildet ist.

Im dargestellten Ausführungsbeispiel sind nur die Spitzen der Injektionsnadeln seitlich herausbewegt. Es ist einleuchtend, dass die Injektionsnadeln über einen längeren Abschnitt herausbewegt werden können, falls gewünscht ist, dass die Injektionsnadeln relativ tief in das Gewebe bzw. in den Muskeln eindringen. Der Durchmesser und die Länge der Injektionsnadeln kann unterschiedlich sein, so dass unterschiedliche Nadelpakete eingesetzt werden können, je nach Patient oder Art der durchzuführenden Behandlung. Eine mögliche Behandlung ist die der Inkontinenz, bei der eine Substanz über die Injektionsnadeln in den Schließmuskel injiziert wird.

Vor einem Zurückziehen des Steuerelementes 50 wird dieses mittels des Steuergriffes 54 um eine Längsachse verdreht, wodurch die Raste von dem zweiten Rastpunkt 73 in den dritten Rastpunkt 74 bewegt wird.

Diese Stellung ist in Fig. 6 dargestellt. Aus dieser Stellung kann nun das Steuerelement 50 wieder nach proximal abgezogen werden, wonach es dann den vierten Rastpunkt 75 erreicht. In dieser Stellung wird dann der Steuergriff 50 in entgegengesetzter Richtung umfänglich verschoben, so dass wieder der ausgängliche Rastpunkt 72 erreicht wird, wobei dies der Stellung von Fig. 4 entspricht.

Beim Bewegen des Steuerelements 50 nach proximal werden die Spitzenabschnitte 48, 48' der Injektionsnadeln 36 bis 39 über die Einziehkeile 66 wieder radial nach innen eingezogen und zwar so weit, dass die Spitzen 41, 41' nicht mehr über die Außenseite des tubusförmigen Körpers 12 hinausreichen, also wieder die Situation von Fig. 4 hergestellt wird.

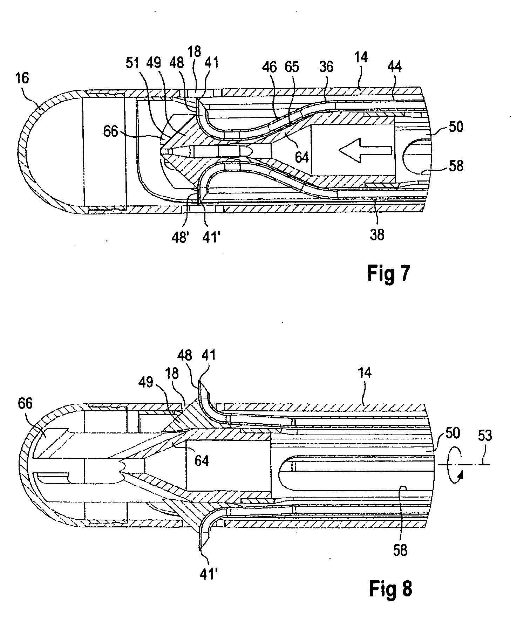

Dieser Bewegungs- und Steuerungsablauf soll nunmehr näher anhand der Schnittdarstellungen von Fig. 7 bis Fig. 12 erläutert werden.

Fig. 7 stellt einen Längsschnitt im distalen Endbereich von Fig. 4 dar, wobei der Schnitt so gelegt ist, dass zwei diametral gegenüberliegende Injektionsnadeln 36 und 38 geschnitten sind. Es ist ersichtlich, dass die Injektionsnadel 36 einen sich geradlinig erstreckenden Abschnitt in einer Form einer Kanüle 44 aufweist, die über eine radial nach innen gerichtete Krümmung 46 in den radial nach außen gebogenen Spitzenabschnitt 48 übergeht, der in der Spitze 41 mündet. Im Bereich des Spitzenabschnittes 48 ist, distal von diesem gesehen, zusätzlich ein Keil 49 montiert, dessen Keilfläche 51 so ausgerichtet ist, dass sie mit der entsprechenden Keilfläche 67 des Einziehkeiles 66 in Berührung treten kann, wie das später noch erläutert wird.

Mittig zwischen die Injektionsnadeln 36 und 38 ist das Steuerelement 50 eingeschoben und zwar derart, dass dessen Spreizkeil 64 bzw. dessen Keilfläche 65 sich an die nach innen gerichtete Krümmung 46 gelegt hat.

Wie zuvor erwähnt, sind die Keilfläche 65 des Spreizkeiles 64 und die Keilfläche 67 des Einziehkeiles 66 umfänglich zueinander versetzt, und zwar um die Umfangsstrecke des Überganges vom zweiten Rastpunkt 73 zum dritten Rastpunkt 74 bzw. des Überganges vom vierten Rastpunkt 75 zum ersten Rastpunkt 72 (siehe Fig. 3).

Daher ist in Fig. 7 ersichtlich, dass der Einziehkeil 66 bzw. dessen Keilfläche hier in dieser Stellung nicht in Eingriff mit der Keilfläche 51 des Keiles 49 steht. Wird nun das Steuerelement 50 nach distal verschoben, wie das in Fig. 7 durch einen Pfeil angedeutet ist, werden die gekrümmten Spitzenabschnitte 48, 48' über den Spreizkeil 64 radial nach außen gedrückt, so dass deren Spitzen 41, 41' über die Außenseite des tubusförmigen Körpers bzw. das Rohr 14 hinaustreten, wie das in Fig. 8 dargestellt ist.

Der Übergang von Fig. 7 zu Fig. 8 entspricht somit dem Übergang von Fig. 4 zu Fig. 5.

Das hier nicht dargestellte Rastelement hat sich dann in der Steuerkurve 70 von dem ersten Rastpunkt 72 zum zweiten Rastpunkt 73 bewegt. Ein weiterer Vorschub des Steuerelementes 50 ist nicht möglich.

Zur Vorbereitung der Einzugsbewegung wird das Steuerelement 50 zunächst um dessen Mittellängsachse 53 gedreht, wie das in Fig. 8 durch einen Pfeil dargestellt ist.

Die Drehbewegung erfolgt, von proximal nach distal gesehen, entgegen dem Uhrzeigersinn und zwar so weit, bis die Raste in dem vierten Rastpunkt 74 eingerastet ist. Diese Drehbewegung entspricht dem Übergang von Fig. 5 zu Fig. 6 bzw. dem Übergang von Fig. 8 zu Fig. 9. In Fig. 9 ist nunmehr ersichtlich, dass jetzt die Keilfläche 67 des Einziehkeiles 66 in Ausrichtung mit der Keilfläche 51 des Keiles 49 steht.

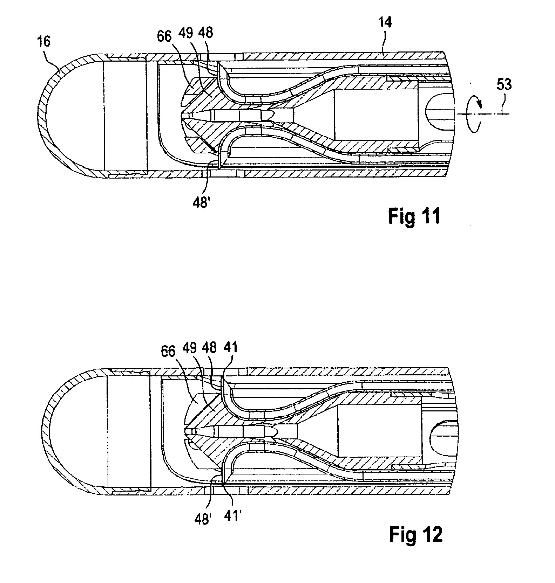

Wird nunmehr das Steuerelement 50 von distal nach proximal gezogen, wobei das über den Steuergriff 54 erfolgt, nähert sich die Keilfläche 67 des Einziehkeiles 66 der zuvor erwähnten Keilfläche 51 des Keiles 49, wie dies in Fig. 10 dargestellt ist und kommt mit dieser in Eingriff. Bei weiterer Bewegung nach proximal, verursacht die Keilfläche 67 des Einziehkeiles 66 ein radial nach innen gerichtetes Einziehen der Spitzenabschnitte 48 bzw. 48' der Injektionsnadeln, wobei der Endzustand in Fig. 11 dargestellt ist.

Aus der Schnittdarstellung von Fig. 11 ist ersichtlich, dass die Spitzenabschnitte 48, 48' vollständig wieder in das Innere des tubusförmigen Körpers 12 eingezogen sind. Dies ist zwangsgesteuert und sichergestellt. In der Stellung von Fig. 6 bzw. 11 ist das Rastelement in dem vierten Rastpunkt 75 der Zwangssteuerung 70 eingerastet. Aus Fig. 11 ist ersichtlich, dass in dieser Drehstellung des Steuerelementes 50, die Keilfläche 67 des Einziehkeiles 66 ein erneutes Spreizen hindern würde. Dazu wird das Steuerelement 50 wieder um dessen Längsachse 53 verdreht, und zwar nunmehr, von proximal nach distal gesehen, im Uhrzeigersinn, wodurch der Übergang von dem vierten Rastpunkt 75 zum ersten Rastpunkt 72 erzielt wird.

Diese Situation ist in Fig. 12 dargestellt, wobei die Situation von Fig. 12 dieselbe Situation ist, wie die von Fig. 7. Somit wäre also die Vorrichtung bereit, einen erneuten Bewegungszyklus durchzuführen.

Je nach Ausgestaltung der Vorrichtung können die in Fig. 2 und 3 dargestellten Teile als Mehrwegteile oder auch als Einwegteile ausgebildet sein. Dadurch entfällt ein Reinigen und Desinfizieren der Kanülen, was bei sehr langen und dünnen Kanülen aufwendig wäre. Man kann auch nur beispielsweise das in Fig. 2 dargestellte Bauteil als Wegwerfteil ausbilden.

Um die Montage und die Lageposition zu erleichtern, kann auch vorgesehen werden, den tubusförmigen Körper 12 aus einem transparenten Material herzustellen und ggf. eine zusätzliche Beleuchtung bereitzustellen oder den stabförmigen Körper von Fig. 3 als Leuchtstab auszubilden, so dass dieser an einer hier nicht dargestellten Leuchtquelle angeschlossen werden kann.

## Patentansprüche

1. Medizinisches Instrument, mit einem tubusförmigen Körper (12), in dem zumindest eine Injektionsnadel (36 - 39) axial unverschiebbar aufgenommen ist, die einen radial nach außen gerichteten Spitzenabschnitt (48, 48') aufweist, und mit einem Steuerelement (50) zum seitlichen Ausfahren zumindest der Spitze (41, 41') der zumindest einen Injektionsnadel (36, 39) aus dem tubusförmigen Körper (12) hinaus, wobei das Steuerelement (50) axial verschiebbar im tubusförmigen Körper (12) aufgenommen ist, und wobei dieses derart mit der zumindest einen Injektionsnadel (36 - 39) in Wirkverbindung steht, dass in einer ersten axialen Verschiebeposition der nach außen gerichtete Spitzenabschnitt (48, 48') der zumindest einen Injektionsnadel (36 - 39) durch das Steuerelement (50) vollständig in den tubusförmigen Körper (12) eingezogen ist, und wobei in einer zweiten axialen Verschiebeposition zumindest die Spitze (41, 41') des nach außen gerichteten Spitzenabschnittes (48, 48') durch das Steuerelement (50) seitlich aus dem tubusförmigen Körper (12) herausgedrückt ist, **dadurch gekennzeichnet, dass** das Steuerelement (50) ein Einziehelement aufweist, das beim axialen Verschieben des Steuerelementes (50) von der zweiten in die erste Position den Spitzenabschnitt (48, 48') der zumindest einen Injektionsnadel (36 - 39) wieder vollständig in den tubusförmigen Körper (12) einzieht.

2. Medizinisches Instrument nach Anspruch 1, **dadurch gekennzeichnet, dass** das Steuerelement (50) ein Spreizelement aufweist, das beim axialen Verschieben des Steuerelements (50) von der ersten in die zweite Verschiebeposition den Spitzenabschnitt (48, 48') der zumindest einen Injektionsnadel (36 - 39) seitlich über den tubusförmigen Körper (12) heraus drückt.

3. Medizinisches Instrument nach Anspruch 2, **dadurch gekennzeichnet, dass** das Spreizelement als Spreizkeil (64) ausgebildet ist.

4. Medizinisches Instrument nach Anspruch 2 oder 3, **dadurch gekennzeichnet, dass** das Einziehelement als Einziehkeil (66) ausgebildet ist.

5. Steuerelement nach einem der Ansprüche 1 bis 4, **dadurch gekennzeichnet, dass** das Steuerelement (50) aus der ersten und der zweiten axialen Verschiebeposition zunächst über eine Drehbewegung in eine verdrehte Position bewegt wird, bevor es in die jeweils andere axiale Verschiebeposition bewegt wird.

6. Medizinisches Instrument nach Anspruch 5, **dadurch gekennzeichnet, dass** die Keilflächen (65, 67) von Spreizkeil (64) und Einziehkeil (66) umfänglich versetzt zueinander sind.

7. Medizinisches Instrument nach Anspruch 5 oder 6, **dadurch gekennzeichnet, dass** eine Zwangssteuerung die Bewegung des Steuerelementes (50) steuert, die eine geschlossene, viereckförmige Steuerbahn (70) aufweist.

8. Medizinisches Instrument nach einem der Ansprüche 1 bis 7, **dadurch gekennzeichnet, dass** im tubusförmigen Körper (12) mehrere Injektionsnadeln (36 - 39) aufgenommen sind, und dass die mehreren Injektionsnadeln (36 - 39) durch ein einziges Steuerelement (50) steuerbar sind.

9. Medizinisches Instrument nach Anspruch 8, **dadurch gekennzeichnet, dass** das Steuerelement (50) Kerben (60 - 63) aufweist, in denen zumindest die nach außen gerichteten Spitzenabschnitte (48, 48') der mehreren Injektionsnadeln (36 - 39) aufnehmbar sind.

10. Medizinisches Instrument nach einem der Ansprüche 1 bis 9, **dadurch gekennzeichnet, dass** die nach außen gerichteten Spitzenabschnitte (48, 48') der Injektionsnadeln (36 - 39) einen ersten Abschnitt (46) aufweisen, der mit dem Spreizelement in Wirkverbindung treten kann und die ferner einen zweiten Abschnitt aufweisen, der mit dem Einziehelement in Wirkverbindung treten kann.

11. Medizinisches Instrument nach Anspruch 10, **dadurch gekennzeichnet, dass** der zweite Abschnitt als Keil (47) ausgebildet ist, der mit dem Einziehkeil (66) beim Einziehen der Injektionsnadeln (36 - 39) in Wirkverbindung steht.

12. Medizinisches Instrument nach einem der Ansprüche 8 bis 11, **dadurch gekennzeichnet, dass** die mehreren Injektionsnadeln (36 - 39) in einem Schutzschaft (30) montiert sind.

13. Medizinisches Instrument nach einem der Ansprüche 1 bis 12, **dadurch gekennzeichnet, dass** das Steuerelement (50) als stabförmiger Körper (52) ausgebildet ist, der distal das Spreizelement und das Einziehelement trägt.

14. Medizinisches Instrument nach Anspruch 13, **dadurch gekennzeichnet, dass** der stabförmige Körper (52) von proximal in einen Schutzschaft (30) zwischen die mehreren Injektionsnadeln (36 - 39) einschiebbar ist.

## Claims

1. Medical instrument comprising a tubular body (12), housing at least one injection needle (36-39) with a radially outwardly directed tip portion (48, 48') in an axially undisplaceable manner, and comprising a control element (50) for deploying at least the tip (41, 41') of the at least one injection needle (36; 39) out sideways from the tubular body (12), wherein said control element (50) is housed in the tubular body (12) in an axially displaceable manner, and wherein said element it in operative connection with the at least one injection needle (36-39) in such a way that, in a first axially displaced position, the outwardly directed tip portion (48, 48') of the at least one injection needle (36-39) is fully retracted in the tubular body (12) by the control element (50) and, in a second axially displaced position, at least the tip (41, 41') of the outwardly directed tip portion (48, 48') is pressed sideways out from the tubular body (12) by the control element (50), **characterized in that** the control element (50) has a retracting element, which fully retracts the tip portion (48, 48') of the at least one injection needle (36-39) back into the tubular body (12) when the control element (50) is axially displaced from the second position into the first position.

2. Medical instrument of Claim 1, **characterized in that** the control element (50) has a spreading element, which presses the tip portion (48, 48') of the at least one injection needle (36-39) out sideways beyond the tubular body (12) when the control element (50) is axially displaced.

3. Medical instrument of Claim 2, **characterized in that** the spreading element is formed as a spreading wedge (64).

4. Medical instrument of Claims 2 or 3, **characterized in that** the retracting element is formed as a retracting wedge (66).

5. Medical instrument of anyone of Claims 1 to 4, **characterized in that** the control element (50) is moved out of the first and second axially displaced positions initially into a twisted position by means of a turning movement before it is moved into the other respective axially displaced position.

6. Medical instrument according to Claim 5, **characterized in that** the wedge surfaces (65, 67) of the wedge part (64) and the retracting part (56) are circumferentially offset in relation to one another.

7. Medical instrument of Claims 5 or 6, **characterized in that** a positive control, which has a closed, rectangular control path (70), controls the movement of the control element (50).

8. Medical instrument of anyone of Claims 1 to 7, **characterized in that** the tubular body (12) houses multiple injection needles (36-39), and **in that** the multiple injection needles (36-39) can be controlled by a single control element (50).

9. Medical instrument of Claim 8, **characterized in that** the control element (50) has notches (60-63), in which at least the outwardly directed tip portions (48, 48') of the multiple injection needles (36-39) can be received.

10. Medical instrument of anyone of Claims 1 to 9, **characterized in that** the outwardly directed tip portions (48, 48') of the injection needles (36-39) have a first portion (46), which can enter into operative connection with the spreading element, and a second portion, which can enter into operative connection with the retracting element.

11. Medical instrument of Claim 10, **characterized in that** the second portion is formed as a wedge (47), which is in operative connection with the retracting wedge (66) when the injection needles (36-39) are retracted.

12. Medical instrument of anyone of Claims 8 to 11, **characterized in that** the multiple injection needles (36-39) are fitted in a protective shaft (30).

13. Medical instrument of anyone of Claims 1 to 12, **characterized in that** the control element (50) is formed as a rod-shaped body (52), which distally bears the spreading element and the retracting element.

14. Medical instrument of Claim 13, **characterized in that** the rod-shaped body (52) can be pushed into the protective shaft (30) between the multiple injection needles (36-39) from the proximal end.

## Revendications

1. Instrument médical comprenant un corps tubulaire (12) dans lequel est logée, sans faculté de coulissement axial, au moins une aiguille d'injection (36-39) munie d'une région pointue (48, 48') dirigée vers l'extérieur dans le sens radial ; et un élément de commande (50) conçu pour faire sortir latéralement, dudit corps tubulaire (12), au moins la pointe (41, 41') de l'aiguille d'injection (36-39) prévue au minimum, ledit élément de commande (50) étant logé à coulissement axial dans ledit corps tubulaire (12) et étant en liaison opérante avec ladite aiguille d'injection (36-39) prévue au minimum, de telle sorte que, en un premier emplacement pris par coulissement axial, la région pointue (48, 48') de ladite aiguille d'injection (36-39) prévue au minimum, dirigée vers l'extérieur, soit intégralement rétractée dans ledit corps tubulaire (12) par ledit élément de commande (50), sachant que, en un second emplacement pris par coulissement axial, au moins la pointe (41, 41') de ladite région pointue (48, 48') dirigée vers l'extérieur est déployée dudit corps tubulaire (12) par ledit élément de commande (50), dans le sens latéral, **caractérisé par le fait que** l'élément de commande (50) présente un élément de rétraction qui, lors du coulissement axial dudit élément de commande (50) depuis le second emplacement jusqu'au premier emplacement, provoque une nouvelle rétraction intégrale, dans ledit corps tubulaire (12), de ladite région pointue (48, 48') de ladite aiguille d'injection (36-39) prévue au minimum.

2. Instrument médical selon la revendication 1, **caractérisé par le fait que** l'élément de commande (50) comporte un élément d'écartement qui, lors du coulissement axial dudit élément de commande (50) depuis le premier emplacement jusqu'au second emplacement pris par coulissement, provoque un déploiement latéral, hors du corps tubulaire (12), de la région pointue (48, 48') de l'aiguille d'injection (36-39) prévue au minimum.

3. Instrument médical selon la revendication 2, **caractérisé par le fait que** l'élément d'écartement est réalisé sous la forme d'un coin d'écartement (64).

4. Instrument médical selon la revendication 2 ou 3, **caractérisé par le fait que** l'élément de rétraction est réalisé sous la forme d'un coin de rétraction (66).

5. Instrument médical selon l'une des revendications 1 à 4, **caractérisé par le fait que**, lorsqu'il quitte les premier et second emplacements pris par coulissement axial, l'élément de commande (50) est tout d'abord animé d'un mouvement rotatoire, jusqu'à un emplacement pris par rotation, avant d'être mû vers l'autre emplacement respectif pris par coulissement axial.

6. Instrument médical selon la revendication 5, **caractérisé par le fait que** les surfaces cunéiformes (65, 67) du coin d'écartement (64) et du coin de rétraction (66) sont mutuellement décalées dans le sens périphérique.

7. Instrument médical selon la revendication 5 ou 6, **caractérisé par le fait qu'**une commande forcée pilote le mouvement de l'élément de commande (50) muni d'une piste de commande (70) fermée, de configuration quadrangulaire.

8. Instrument médical selon l'une des revendications 1 à 7, **caractérisé par le fait que** le corps tubulaire (12) renferme plusieurs aiguilles d'injection (36-39) ; et **par le fait que** les multiples aiguilles d'injection (36-39) peuvent être commandées par le biais d'un unique élément de commande (50).

9. Instrument médical selon la revendication 8, **caractérisé par le fait que** l'élément de commande (50) présente des entailles (60-63) aptes à recevoir au moins les régions pointues (48, 48'), dirigées vers l'extérieur, des multiples aiguilles d'injection (36-39).

10. Instrument médical selon l'une des revendications 1 à 9, **caractérisé par le fait que** les régions pointues (48, 48') des aiguilles d'injection (36-39), dirigées vers l'extérieur, comprennent un premier tronçon (46) pouvant venir en liaison opérante avec l'élément d'écartement et, par ailleurs, un second tronçon pouvant venir en liaison opérante avec l'élément de rétraction.

11. Instrument médical selon la revendication 10, **caractérisé par le fait que** le second tronçon est réalisé sous la forme d'un coin (47) qui, lors de la rétraction des aiguilles d'injection (36-39), se trouve en liaison opérante avec le coin de rétraction (66).

12. Instrument médical selon l'une des revendications 8 à 11, **caractérisé par le fait que** les multiples aiguilles d'injection (36-39) sont montées dans un fût protecteur (30).

13. Instrument médical selon l'une des revendications 1 à 12, **caractérisé par le fait que** l'élément de commande (50) se présente comme un corps (52) en forme de tige, portant l'élément d'écartement et l'élément de rétraction dans la région distale.

14. Instrument médical selon la revendication 13, **caractérisé par le fait que** le corps (52) en forme de tige peut être inséré dans un fût protecteur (30), entre les multiples aiguilles d'injection (36-39), à partir de la région proximale.
